# EUROPEAN PATENT SPECIFICATION

(11) **EP 0 922 044 B1**
(45) Date of publication and mention of the grant of the patent: **10.10.2001**
(21) Application number: 97936632.5
(22) Date of filing: 04.07.1997
(51) Int. Cl.: C07D 471/04, A61K 31/47

(54) **PYRROLO(3,2-C) QUINOLINE DERIVATIVES**
PYRROLO(3,2-C)CHINOLIN-DERIVATE
DERIVES DE LA PYRROLO 3,2-C] QUINOLINE

(30) Priority: 05.08.1996 GB 9616467
(43) Date of publication of application: 16.06.1999
(73) Proprietor: PHARMACIA & UPJOHN S.p.A., 20152 Milano (IT)
(72) Inventor: PEVARELLO, Paolo, I-27100 Pavia (IT); HEIDEMPERGHER, Franco, I-20015 Parabiago (IT); DELLA TORRE, Arturo, I-21013 Gallarate (IT); VARASI, Mario, I-20142 Milano (IT); SPECIALE, Carmela, I-20014 Nerviano (IT)
(86) International application number: EP9703639
(87) International publication number: WO9805660

(56) References cited:
- EP-A- 0 430 485
- EP-A- 0 555 149
- US-A- 5 605 906
- CHEMICAL ABSTRACTS, vol. 121, no. 23, 5 December 1994 Columbus, Ohio, US; abstract no. 271932v, XP002047291 & F.E. JENSEN ET AL.: NEUROSCIENCE (OXFORD), vol. 62, no. 2, 1994, pages 399-406,
- CHEMICAL ABSTRACTS, vol. 85, no. 19, 8 November 1976 Columbus, Ohio, US; abstract no. 141698a, XP002047292 & Y. SHIBATA ET AL.: ACTA VITAMINOL. ENZYMOL., vol. 29, no. 1-6, 1975, pages 190-193,
- PATENT ABSTRACTS OF JAPAN vol. 012, no. 426 (C-542), 10 November 1988 & JP 63 156724 A (SOGO YATSUKOU K.K.)

## Description

The present invention relates to pyrrolo[3,2-c]quinoline derivatives, to a process for their preparation, to pharmaceutical compositions containing them and to their use for the prevention and/or treatment of neurodegenerative diseases, such as cerebral ischemia/hypoxia, Parkinson's disease, epilepsy, Huntington's chorea, Alzheimer's and non-Alzheimer's dementias (AIDS, infarctual dementia), head and spinal cord injury, Amyotrophic Lateral Sclerosis, glaucoma/retinopathy, infections and inflammations of the brain.

The compounds of the invention act as inhibitors of Kynurenine-3-hydroxylase (KYN-OH), the enzyme which forms part of the metabolic pathway of kynurenine.

It is well known that through the kynurenine pathway, tryptophan metabolism gives rise to the formation of quinolinic acid (QUIN) on the one side and kynurenic acid (KYNA) on the other as shown in the following diagram

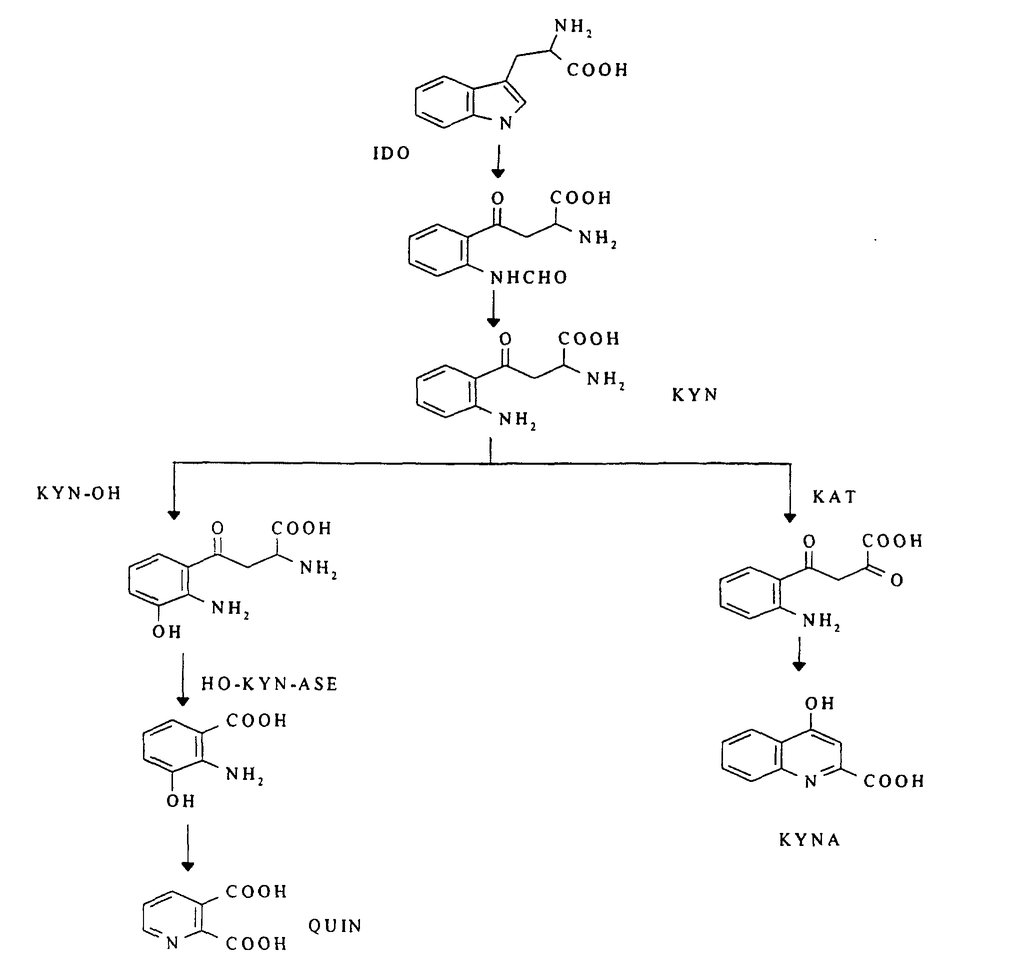

KYNA is endowed with neuroprotective properties (J. Neurosci. 1990,10,2965-2973), whereas QUIN is a relatively potent neurotoxin which has been implicated in the pathogenesis of a variety of neurological disorders including Huntington's disease and epilepsy(Life Sci.1984,35,19-32;Nature, 1986,321, 168-171;Science,1983,219,316-318). Increased concentrations of QUIN have also been indicated as responsible for neurological disorders accompanying many infections and inflammatory diseases including Aquired Immunodeficiency Syndrome (AIDS) (Ann. Neurol. 1991,29,202-209).

One of the main strategies aimed at altering the KYNA/QUIN balance blocking QUIN's production and increasing KYNA production, is inhibiting key enzymes of the KYN pathway, among which Kynurenine-3-hydroxylase is of primary importance.

Consequently, compounds capable of inhibiting this enzyme would be useful in the prevention and treatment of all the pathologies involving quinolinic acid, or excessive activation of neurotransmission mediated by excitatory amino acid receptors.

Accordingly, the present invention provides a compound of the following formula (I). 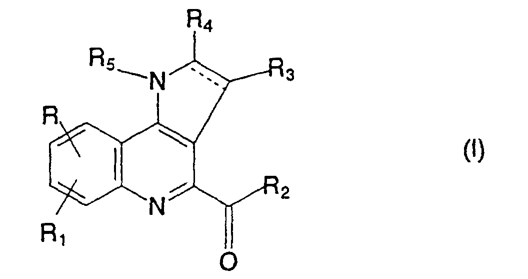 wherein
each of R and R₁, which are the same or different, is hydrogen, halogen, hydroxy, trifluoromethyl, cyano, nitro, phenyl, benzyl, C₁-C₆ alkyl, C₁-C₆ alkoxy, C₁-C₆ alkylthio, SOR₆, SO₂R₆, SO₂NH₂, formyl, C₂-C₆ alkanoyl, carboxy, C₁-C₆ alkoxycarbonyl or a -N(R₇R₈) group in which each of R₇ and R₈, which are the same or different, is hydrogen, C₁-C₆ alkyl, formyl or C₂-C₆ alkanoyl;

R₂ is hydroxy, C₁-C₆ alkoxy, -N(R₉R₁₀) in which each of R₉ and R₁₀, which are the same or different, is hydrogen, C₁-C₆ alkyl or phenyl, or NHOR₁₁ wherein R₁₁ is hydrogen, C₁-C₆ alkyl, benzyl or phenyl;
each of R₃, R₄ and R₆, which are the same or different, is hydrogen, C₁-C₆ alkyl, benzyl or phenyl; and R₅ is hydrogen, C₁-C₆ alkyl, benzyl, C₂-C₆ alkanoyl, C₂-C₄ alkenyl, C₂-C₄ alkynyl or formyl; or a pharmaceutically or veterinarily acceptable salt thereof.

The alkyl and alkoxy groups may be branched or straight-chain groups. Representative examples of C₁-C₆ alkyl groups include methyl, ethyl, n- and iso-propyl, n-, iso-, sec- and tert-butyl. Representative examples of C₁-C₆ alkoxy groups include methoxy and ethoxy. A halogen may be fluorine, bromine, chlorine or iodine, in particular chlorine or bromine.

The symbol ---- represents a single or double bond.

The pharmaceutically acceptable salts of the compounds of formula (I) include the salts of inorganic bases, for example hydroxides of alkaline metals, e.g. sodium or potassium, or alkaline-earth metals, e.g. calcium or magnesium, and the salts of organic bases, such as for example aliphatic amines, e.g. methylamine, ethylamine, diethylamine, trimethylamine, or heterocyclic amines, e.g. piperidine.

The present invention also include within its scope all the possible isomers and their mixtures and both the metabolites and the pharmaceutically acceptable bio-precursors (otherwise known as pro-drugs) of the compounds of formula (I). Preferred compounds of the invention are the compounds of formula (I) wherein:
each of R and R₁, which are the same or different, is hydrogen or halogen, hydroxy, trifluoromethyl, cyano, nitro, C₁-C₄ alkyl, C₁-C₄ alkoxy, C₁-C₄ alkylthio, SOR₆, SO₂R₆, SO₂NH₂, formyl, C₂-C₆ alkanoyl, carboxy, C₁-C₆ alkoxycarbonyl or -N(R₇R₈) group in which each of R₇ and R₈, which are the same or different, is hydrogen, C₁-C₄ alkyl or formyl;
R₂ is hydroxy, C₁-C₄ alkoxy, -N(R₉R₁₀) in which each of R₉ and R₁₀, which may be the same or different, is hydrogen, C₁-C₄ alkyl or phenyl; NHOR₁₁, wherein R₁₁ is hydrogen, C₁-C₄ alkyl, benzyl or phenyl;
each of R₃, R₄ and R₆, which are the same or different, is hydrogen, C₁-C₄ alkyl or phenyl;
R₅ is hydrogen or C₁-C₄ alkyl; and
the symbol ---- represents a double bond;
and the pharmaceutically acceptable salt thereof.

Examples of preferred compounds of the invention are the following:
7-chloro-3-methyl-1H-pyrrolo[3,2-c]quinoline-4-carboxylic acid;
7-chloro-1,3-dimethyl-1H-pyrrolo[3,2-c]quinoline-4-carboxylic acid;
8-chloro-3-methyl-1H-pyrrolo[3,2-c]quinoline-4-carboxylic acid;
3-methyl-1H-pyrrolo[3,2-c]quinoline-4-carboxylic acid;
6-chloro-3-methyl-1H-pyrrolo[3,2-c]quinoline-4-carboxylic acid;
9-chloro-3-methyl-1H-pyrrolo[3,2-c]quinoline-4-carboxylic acid;
6,7-dichloro-3-methyl-1H-pyrrolo[3,2-c]quinoline-4-carboxylic acid;
7,9-dichloro-3-methyl-1H-pyrrolo[3,2-c]quinoline-4-carboxylic acid;
7,8-dichloro-3-methyl-1H-pyrrolo[3,2-c]quinoline-4-carboxylic acid;
7-chloro-1H-pyrrolo[3,2-c]quinoline-4-carboxylic acid;
7-chloro-3-ethyl-1H-pyrrolo[3,2-c]quinoline-4-carboxylic acid;
7-chlaro-3-phenyl-1H-pyrrolo-[3,2-c]quinoline-4-carboxylic acid;
7-fluoro-3-methyl-1H-pyrrolo[3,2-c]quinoline-4-carboxylic acid;
8-fluoro-3-methyl-1H-pyrrolo[3,2-c]quinoline-4-carboxylic acid;
6-fluoro-3-methyl-1H-pyrrolo[3,2-c]quinoline-4-carboxylic acid;
9-fluoro-3-methyl-1H-pyrrolo[3,2-c]quinoline-4-carboxylic acid;
6,7-difluoro-3-methyl-1H-pyrrolo[3,2-c]quinoline-4-carboxylic acid;
7,9-difluoro-3-methyl-1H-pyrrolo[3,2-c]quinoline-4-carboxylic acid;
7,8-difluoro-3-methyl-1H-pyrrolo[3,2-c]quinoline-4-carboxylic acid;
7-bromo-3-methyl-1H-pyrrolo[3,2-c]quinoline-4-carboxylic acid;
8-bromo-3-methyl-1H-pyrrolo[3,2-c]quinoline-4-carboxylic acid;
6,7-dibromo-3-methyl-1H-pyrrolo[3,2-c]quinoline-4-carboxylic acid;
7,8-dibromo-3-methyl-1H-pyrrolo[3,2-c]quinoline-4-carboxylic acid;
7-nitro-3-methyl-1H-pyrrolo[3,2-c]quinoline-4-carboxylic acid;
8-nitro-3-methyl-1H-pyrrolo[3,2-c]quinoline-4-carboxylic acid;
and the pharmaceutically acceptable salts thereof.

The compounds of the invention and the salts thereof can be obtained by a process which comprises hydrolysing a compound of formula (II) 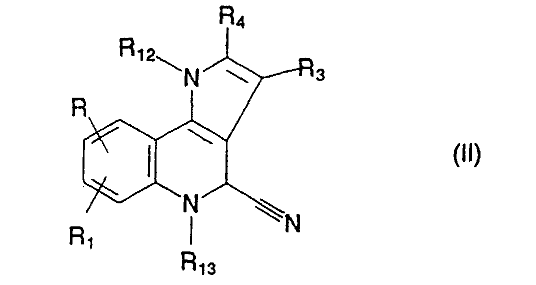 wherein R, R₁ R₃ and R₄ are as defined above, R₁₂ is C₂-C₄ alkanoyl and R₁₃ is C₂-C₄ alkanoyl or benzoyl, and, if desired, converting a compound of formula (I) into another compound of formula (I), and/or converting a compound of formula (I) into a pharmaceutically acceptable salt thereof, and/or, if desired, converting a salt into a free compound.

R₁₂ in formula (II) is preferably an acetyl or propionyl group.

The hydrolysis of a compound of formula (II) to obtain a compound of formula (I) wherein R₂ is OH and R₅ is H can be carried out according to well known methods in the art. For instance the reaction can be performed in an aqueous solution of a halohydric acid, typically HBr, in a suitable solvent, e.g. acetic acid, at a temperature from 90°C to reflux.

A compound of formula (I) can be converted into another compound of formula (I) according to known methods. Thus, for instance, a compound of formula (I), wherein R₂ is hydroxy, can be converted into another compound of formula (I) wherein R₂ is C₁-C₄ alkoxy by conventional alkylating methods, e.g. by treatment with a suitable alkylating agent, typically a C₁-C₆ alkyl halide wherein the halide is preferably an iodide, in the presence of a base, e.g. potassium bicarbonate, in a suitable solvent, e.g. dimethylformamide at a temperature ranging from about 0°C to about 60°C. A compound of formula (I) wherein R₅ is hydrogen may be converted into another compound of formula (I) wherein R₅ is C₁-C₆ alkyl by treatment with an alkylating agent in the presence of a strong base in a suitable solvent at a temperature of from room temperature to about 100°C. The alkylating agent is typically a C₁-C₆ alkyl halide, preferably a C₁-C₆ alkyl iodide. The strong base is typically potassium hydroxide. The solvent is typically dimethylformamide or dimethylsulphoxide. A compound of formula (I) wherein R₂ is C₁-C₄ alkoxy may be converted into another compound of formula (I) wherein R₂ is hydroxy, by conventional hydrolytic methods, e.g. basic hydrolysis, using for example sodium hydroxide or potassium hydroxide, in a solvent such as water or a lower aliphatic alcohol and operating at a temperature ranging from room temperature to about 90°C and then acidifying; or acid hydrolysis, for instance using an aqueous solution of a halohydric acid, typically HCl in a suitable solvent, e.g. acetic acid, at a temperature ranging from 90°C to reflux temperature. A compound of formula (I), wherein R₂ is hydroxy, may be converted into another compound of formula (I), wherein R₂ is -N(R₉R₁₀) by conventional methods, e.g. methods employed usually in the chemistry of peptides. Furthermore, a compound of formula (I) wherein one or more of R or R₁ is nitro may be converted in a compound of formula (I) wherein one or more of R and R₁ is amino by known procedures, e.g. by catalytic hydrogenation, preferably using PtO₂ as a catalyst. Alkylation of a compound of formula (I) wherein one or more of R and R₁ is amino may be carried out for example by reaction of a suitable C₁-C₄ aldehyde under reductive amination conditions, e.g. in the presence of a suitable complex hydride such as sodium borohydride or sodium cyanoborohydride, in a suitable solvent such as methanol or acetonitrile, at a suitable temperature, e.g from -20°C to reflux, for a suitable time, e.g. from 1 to 48 hours. Said alkylation process provides a mixture of N,N'-dialkylated compounds of formula (I). The single alkylated compounds may be separated from the mixture according to well known methods, e.g. by silica gel column chromatography. The optional salification of a compound of formula (I) as well as the conversion of a salt into the free compound and the separation of a mixture of isomers into the single isomers may be carried out by conventional methods.

The compounds of formula (II), which are new and are a further object of this invention, can be obtained by treating a compound of formula (III) 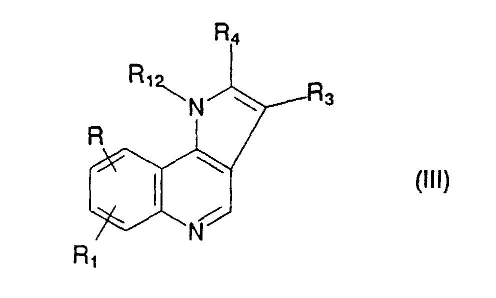 wherein R, R₁, R₃, R₄ and R₁₂ are as defined above, with a cyanide salt and an acyl halide.

The reaction is typically conducted in anhydrous benzene or methylene chloride, or in a mixture of solvents, at a temperature from about 0°C to room temperature. This is the well-known Reissert reaction (see, for instance, Adv. Heterocycl. Chem. 1979, 24, pp 127-214 and 1968, 9, pp 1-29). The cyanide salt may be, for example, sodium cyanide, potassium cyanide or silver cyanide. The preferred mixture of solvents comprises water and methylene chloride. A phase transfer catalyst may optionally be present. The acyl halide is preferably benzoyl chloride.

A compound of formula (I) can also be obtained by a process which comprises
(i) converting a compound of formula (III), as defined above, to its N-oxide derivative;
(ii) treating the resulting N-oxide derivative with an excess of trimethylsilyl cyanide in the presence of a base; and
(iii) hydrolysing the compound produced in step (ii).

The conversion to an N-oxide derivative in step (I) is a conventional process performed under conventional conditions. In step (ii) the amount of trimethylsilyl cyanide used is typically from 2 to 5 equivalents. The base may be, for instance, triethylamine. The reaction of step (ii) is typically performed in a suitable solvent such as dimethyl formamide, at a temperature from room temperature to reflux, for a time of from 4 to 72 hours. The hydrolysis of step (iii) is performed under conventional conditions.

Compounds of formula (III) can be obtained by reacting a compound of formula (IV) 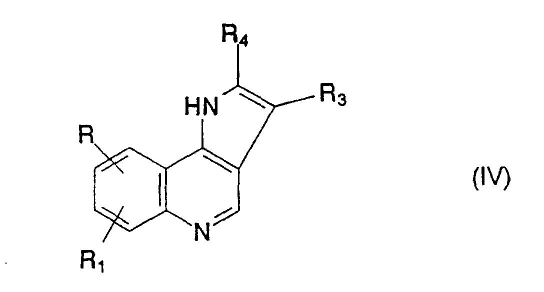 wherein R, R₁, R₃ and R₄ are as defined above, with a compound of formula (V) or (VI):

R₁₂X (V)

(R₁₂)₂O (VI)

wherein R₁₂ is C₂-C₄ alkanoyl and X is a halogen. R₁₂ is preferably acetyl or propionyl and the halogen is preferably chlorine or bromine. A compound of formula (VI) is preferably acetic or propionic anhydride. This reaction can be carried out, for example, in the presence of a base such as triethylamine, in a solvent such as methylene chloride, at a temperature varying between 0°C and room temperature. When in the compounds of the present invention and in the intermediate compounds (II) to (IV) groups are present which need to be protected before submitting them to the hereabove illustrated reactions, they may be protected before being reacted and then deprotected according to methods well known in organic chemistry. The compounds of formula (IV) are either known or may be prepared according to known methods, e.g. as described in J. Heterocyclic Chem. 1978, 15, 913. The compounds of formula (V) are well known compounds in organic chemistry.

### Pharmacology

The efficacy of the compounds of the invention in the inhibition of the enzyme kynurenine-3-hydroxylase has been evaluated in rat liver mitochondrial extract as following reported, accrding to known methods (A radiometric Assay for Kynurenine 3-Hydroxylase Based on the Release of ³H₂O during Hydroxylation of L-(3,5-³H)Kynurenine; Joel B. Erickson, Ellen M. Flanagan, Suzanne Russo, and John F. Reinhardt Jr.; Analytical Biochem (1992), 205, 257-262) with minor modifications.

### Kynurenine-3-hydroxylase assay in rat liver

The assay for kynurenine 3-hydroxylase was based on enzymatic synthesis of tritiated water during hydroxylation reaction. Radiolabelled water was quantified following selective adsorption of the isotopic substrate and its metabolites with active charcoal.

Rat liver mitochondrial extract was used as enzymatic preparation for this assay.

The assay for kynurenine 3-hydroxylase activity was carried out at 37°C for a time of 30 min. The reaction mixture of a total volume of 100 ml was constituted of 44 mg of suspended extract, 100 mM Tris/Cl buffer pH 8.1, 10 mM EDTA, 100mM KCl, 0.8 mM NADPH, 0.025 mM L-kynurenine, 0.5 mCi L-(3,5-³H)Kynurenine (10 Ci/mmol) and 10 ml of different concentration of inhibitor solutions. After the incubation the reaction was terminated by the addition of 1 mL of 7.5% (w/v) activated charcoal, vortexed and centrifugated for 7 min.

A 500 aliquot of supernatant was counted by scintillation spectroscopy in 5 ml of liquid scintillation.

The efficacy of the compounds of the invention in the inhibition of the enzyme kynurenine-3-hydroxylase has been evaluated in rat brain homogenate by the conversion of L-kynurenine to L-3-hydroxykynurenine according to the method described below.

### Kynurenine-3-hydroxylase assay in the rat brain

Brain was homogenized in ice-cold 0.32 M sucrose and centrifuged at 12000 x g for 30 min at 4°C. The pellet was washed three times with 0.32 M sucrose by centrifugation and suspended in 0.14 M KCl in 20 mM K-phosphate buffer at pH 7 (1 g brain in 2 ml buffer).

The reaction mixture contained: 75 *µ*l of suspended homogenate; 100*µ*l of 2 mM MgCl₂, 0.4 mM NADPH,50 *µ*M L-kynurenine in 50 mM-K-phosphate buffer pH 7.5 and 25 *µ*l of different concentrations of the tested compound. The reaction was terminated by addition of 200 *µ* l of 1 M HClO₄ after 60 min incubation at 37°C. L-3-hydroxykynurenine formed was quantified by HPLC with coloumetric detection at working voltage of +0.2 V. The column was a 10 cm C 18 reversed phase (3 *µ*m particulate). The mobile phase consisted of 950 ml distilled water, 20 ml acetonitrile, 9 ml triethylamine, 5.9 ml phosphoric acid, 100 mg sodium Na EDTA and 1.5 g heptanesulfonic acid. The flow rate was 1 ml/min.

### Biological results

The obtained results, which have been reported in the following Table 1 demonstrate the efficacy of the representative compounds of the invention.

**Table 1**

| KYN-3-OH inhibition (IC₅₀) | | |
|---|---|---|
| Compound | Liver | Brain |
| FCE 27605 | 24 *µ*M | 11 *µ*M |
| FCE 29370 | 61 *µ*M | - |
| FCE 29464 | 34 *µ*M | - |

FCE 27605 is the code for 7-chloro-3-methyl-1H-pyrrolo[3,2-c] quinoline-4-carboxylic acid;
FCE 29370 is the code for 8-chloro-3-methyl-1H-pyrrolo[3,2-c] quinoline-4-carboxylic acid;
FCE 29464 is the code for 7-chloro-3-phenyl-1H-pyrrolo[3,2-c] quinoline-4-carboxylic acid.

A human or animal, e.g. a mammal, may thus be treated by a method which comprises the administration thereto of a compound of formula (I) or a pharmaceutcially or veterinarily acceptable salt thereof. The condition of a patient in need of a kynurenine-3-hydroxylase (KYN-OH) inhibitor may thereby be improved. The compounds and salts of the invention may be used in particular in the prevention and/or treatment of cerebral ischemia/hypoxia, Parkinson's disease, epilepsy, Huntington's chorea, Alzheimer's or non-Alzheimer's dementia, head or spinal cord injury, Amyotrophic Lateral Sclerosis, glaucoma/retinopathy, or infections or inflammations of the brain.

The dosage level suitable for administration to adult humans depends on the age, weight, conditions of the patient and on the administration route; for example, the dosage adopted for oral administration e.g. for the representative compound of the invention FCE 27605 may range from about 10 to about 500 mg pro dose, from 1 to 5 times daily.

The compounds of the invention can be administered in a variety of dosage forms, e.g. orally, in the form of tablets, capsules, sugar or film coated tablets, liquid solutions or suspensions; rectally in the form of suppositories; parenterally, e.g. intramuscularly, or by intravenous injection or infusion.

The invention includes pharmaceutical compositions comprising a compound of the invention in association with a pharmaceutically acceptable excipient (which can be a carrier or a diluent).

The pharmaceutical compositions containing the compounds of the invention are usually prepared following conventional methods and are administered in a pharmaceutically suitable form.

For example, the solid oral forms may contain, together with the active compound, diluents, e.g. lactose, dextrose, saccharose, sucrose, cellulose, corn starch or potato starch; lubricants, e.g. silica, talc, stearic acid, magnesium or calcium stearate, and/or polyethylene glycols; binding agents, e.g. starches, arabic gum, gelatin, methylcellulose, carboxymethylcellulose or polyvinyl pyrrolidone; disaggregating agents, e.g. a starch, alginic acid, alginates or sodium starch glycolate; effervescing mixtures; dyestuffs; sweeteners; wetting agents such as lecithin, polysorbates, laurylsulphates; and, in general, non-toxic and pharmacologically inactive substances used in pharmaceutical formulations. Said pharmaceutical preparations may be manufactured in known manner, for example, by means of mixing, granulating, tabletting, sugar-coating, or filmcoating processes.

The liquid dispersions for oral administration may be e.g. syrups, emulsions and suspensions.

The syrups may contain as carrier, for example, saccharose or saccharose with glycerine and/or mannitol and/or sorbitol. The suspensions and the emulsions may contain as carrier, for example, a natural gum, agar, sodium alginate, pectin, methylcellulose, carboxymethylcellulose, or polyvinyl alcohol.

The suspension or solutions for intramuscolar injections may contain, together with the active compound, a pharmaceutically acceptable carrier, e.g. sterile water, olive oil, ethyl oleate, glycols, e.g. propylene glycol, and, if desidered, a suitable amount of lidocaine hydrochloride. The solutions for intravenous injections or infusions may contain as carrier, for example, sterile water or preferably they may be in the form of sterile, acqueous, isotonic saline solutions.

The suppositories may contain together with the active compound a pharmaceutically acceptable carrier, e.g. cocoa butter, polyethylene glycol, a polyoxyethylene sorbitan fatty acid ester surfactant or lecithin.

The following examples illustrate but do not limit the invention.

### Example 1

### Preparation of 1-(7-chloro-3-methyl-pyrrolo[3,2-c] quinolin-1-yl)-ethanone.

A solution of 7-chloro-3-methyl-1H-pyrrolo[3,2-c]quinoline (5 g; 0.023 mol) in 50 ml of acetic anhydride was refluxed 4 hours under a nitrogen atmosphere. The mixture was cooled to room temperature, poured into crushed ice and allowed to stand overnight. The crude solid so obtained was collected by filtration and crystallized from absolute ethanol to yield 4.5 g of the desired product as a light brown solid (m.p. 159-160.5°C). C₁₄H₁₁ClN₂O (required: C(64.99); H(4.29); Cl(13.71); N (10.83); found: C(64.91); H(4.30); Cl (13.69); N (10.80).

Analogously the following products can be prepared :
1-(8-chloro-3-methyl-pyrrolo[3,2-c]quinolin-1-yl)-ethanone; m.p. 198°-201°C;
1-(3-methyl-pyrrolo[3,2-c]quinolin-1-yl)-ethanone;
1-(6-chloro-3-methyl-pyrrolo[3,2-c]quinolin-1-yl)-ethanone,
1-(9-chloro-3-methyl-pyrrolo[3,2-c]quinolin-1-yl)-ethanone;
1-(6,7-dichloro-3-methyl-pyrrolo[3,2-c]quinolin-1-yl)-ethanone;
1-(7,9-dichloro-3-methyl-pyrrolo[3,2-c]quinolin-1-yl)-ethanone;
1-(7,8-dichloro-3-methyl-pyrrolo[3,2-c]quinolin-1-yl)-ethanone;
1-(7-chloro-3-ethyl-pyrrolo[3,2-c]quinolin-1-yl)-ethanone;
1-(7-chloro-pyrrolo[3,2-c]quinolin-1-yl)-ethanone;
1-(7-fluoro-3-methyl-pyrrolo[3,2-c]quinolin-1-yl)-ethanone; m.p. 150°-152° C;
1-(8-fluoro-3-methyl-pyrrolo[3,2-c]quinolin-1-yl)-ethanone;
1-(6-fluoro-3-methyl-pyrrolo[3,2-c]quinolin-1-yl)-ethanone;
1-(9-fluoro-3-methyl-pyrrolo[3,2-c]quinolin-1-yl)-ethanone;
1-(6,7-difluoro-3-methyl-pyrrolo[3,2-c]quinolin-1-yl)-ethanone; 1-(7,9-difluoro-3-methyl-pyrrolo[3,2-c]quinolin-1-yl) -ethanone;
1-(7,8-difluoro-3-methyl-pyrrolo[3,2-c]quinolin-1-yl)-ethanone;
1-(7-bromo-3-methyl-pyrrolo[3,2-c]quinolin-1-yl)-ethanone;
1-(6,7-dibromo-3-methyl-pyrrolo[3,2-c]quinolin-1-yl)-ethanone;
1-(7,8-dibromo-3-methyl-pyrrolo[3,2-c]quinolin-1-yl)-ethanone;
1-(7-nitro-3-methyl-pyrrolo[3,2-c]quinolin-1-yl)-ethanone;
1-(8-nitro-3-methyl-pyrrolo[3,2-c]quinolin-1-yl)-ethanone;
1-(7-chloro-3-phenyl-pyrrolo[3,2-c]quinolin-1-yl)-ethanone m.p. 149-150°C (dec.);

### Example 2

### Preparation of 1-acetyl-5-benzoyl-7-chloro-3-methyl-4,5-dihydro-1H-pyrrolo[3,2-c]quinoline-4-carbonitrile

To a vigorously stirred mixture of 1-(7-chloro-3-methylpyrrolo[3,2-c]quinolin-1-yl)-ethanone (6.3 g; 0.0243 mol) and potassium cyanide (4.73 g; 0.0726 mol) in 80 ml of methylene chloride and 30 ml of water, benzoyl chloride (5.64 ml; 0.0486 mol) was dropped within 2 hours at room temperature. The mixture was stirred for 20 hours, then diluted with water and the two phases separated. The aqueous layer was extracted with methylene chloride, then the combined organic layers were washed with brine, dried over anhydrous sodium sulfate and evaporated to dryness. The residue was purified by silica gel flash-chromatography (eluant: cyclohexane-ethyl acetate, 2:1) to give the desired product as a light brown solid (m.p. 196-201 °C); C₂₂H₁₆ClN₃O₂: required: C (67.77); H (4.13), Cl (9.09); N (10.78); found: C(67.36); H (4.48); Cl (9.24); N (10.39).

Analogously the following products can be prepared :
1-acetyl-5-benzoyl-8-chloro-3-methyl-4,5-dihydro-1H-pyrrolo[3,2-c]quinoline-4-carbonitrile; m.p. 190°C (dec.) 1-acetyl-5-benzoyl-3-methyl-4,5-dihydro-1H-pyrrolo [3,2-c]quinoline-4-carbonitrile;
1-acetyl-5-benzoyl-6-chloro-3-methyl-4,5-dihydro-1H-pyrrolo[3,2-c]quinoline-4-carbonitrile;
1-acetyl-5-benzoyl-9-chloro-3-methyl-4,5-dihydro-1H-pyrrolo[3,2-c]quinoline-4-carbonitrile;
1-acetyl-5-benzoyl-6,7-dichloro-3-methyl-4,5-dihydro-1H-pyrrolo[3,2-c]quinoline-4-carbonitrile;
1-acetyl-5-benzoyl-7,8-dichloro-3-methyl-4,5-dihydro-1H-pyrrolo[3,2-c]quinoline-4-carbonitrile;
1-acetyl-5-benzoyl-7,9-dichloro-3-methyl-4,5-dihydro-1H-pyrrolo[3,2-c]quinoline-4-carbonitrile;
1-acetyl-5-benzoyl-7-chloro-3-ethyl-4,5-dihydro-1H-pyrrolo[3,2-c]quinoline-4-carbonitrile;
1-acetyl-5-benzoyl-7-chloro-4,5-dihydro-1H-pyrrolo [3,2-c]quinoline-4-carbonitrile;
1-acetyl-5-benzoyl-8-fluoro-3-methyl-4,5-dihydro-1H-pyrrolo[3,2-c]quinoline-4-carbonitrile;
1-acetyl-5-benzoyl-6-fluoro-3-methyl-4,5-dihydro-1H-pyrrolo[3,2-c]quinoline-4-carbonitrile;
1-acetyl-5-benzoyl-9-fluoro-3-methyl-4,5-dihydro-1H-pyrrolo[3,2-c]quinoline-4-carbonitrile;
1-acetyl-5-benzoyl-6,7-difluoro-3-methyl-4,5-dihydro-1H-pyrrolo[3,2-c]quinoline-4-carbonitrile;
1-acetyl-5-benzoyl-7,9-difluoro-3-methyl-4,5-dihydro-1H-pyrrolo[3,2-c]quinoline-4-carbonitrile;
1-acetyl-5-benzoyl-7,8-difluoro-3-methyl-4,5-dihydro-1H-pyrrolo[3,2-c]quinoline-4-carbonitrile;
1-acetyl-5-benzoyl-7-bromo-3-methyl-4,5-dihydro-1H-pyrrolo[3,2-c]quinoline-4-carbonitrile;
1-acetyl-5-benzoyl-6,7-dibromo-3-methyl-4,5-dihydro-1H-pyrrolo[3,2-c]quinoline-4-carbonitrile;
1-acetyl-5-benzoyl-7,8-dibromo-3-methyl-4,5-dihydro-1H-pyrrolo[3,2-c]quinoline-4-carbonitrile;
1-acetyl-5-benzoyl-7-nitro-3-methyl-4,5-dihydro-1H-pyrrolo[3,2-c}quinoline-4-carbonitrile;
1-acetyl-5-benzoyl-8-nitro-3-methyl-4,5-dihydro-1H-pyrrolo[3,2-c]quinoline-4-carbonitrile;
1-acetyl-5-benzoyl-7-fluoro-3-methyl-4,5-dihydro-1H-pyrrolo[3,2-c]quinoline-4-carbonitrile; m.p.85°C (dec);
1-acetyl-5-benzoyl-7-chloro-3-phenyl-4,5-dihydro-1H-pyrrolo [3,2-c]quinoline-4-carbonitrile; m.p. 90°C (dec.)

### Example 3

### Preparation of 7-chloro-3-methyl-1H-pyrrolo[3,2-c]quinoline-4-carboxylic acid.

A solution of 1-acetyl-5-benzoyl-7-chloro-3-methyl-4,5-dihydro-1H-pyrrolo[3,2-c]quinoline-4-carbonitrile (4.9 g; 0.0126 mol) in acetic acid (35 ml) and 48% hydrobromic acid (35 ml) was heated at 100°C for 1 hour. The solution was evaporated to dryness and the residue crystallized from ethanol/water 1/l to obtain a light brown solid (m.p. 300 °C (dec.). C₁₃H₉ClN₂O₂; required: C (59.89); H (3.48); Cl (13.60); N (10.74); found: C (59.5); H (3.66); Cl (13.74); N (10.41). Analogously the following products can be prepared :
8-chloro-3-methyl-1H-pyrrolo[3,2-c]quinoline-4-carboxylic acid; m.p. 230°C (dec.);
3-methyl-1H-pyrrolo[3,2-c]quinoline-4-carboxylic acid, hydrate; m.p. 230°C (dec.);
6-chloro-3-methyl-1H-pyrrolo[3,2-c]quinoline-4-carboxylic acid;
9-chloro-3-methyl-1H-pyrrolo[3,2-c]quinoline-4-carboxylic acid;
6,7-dichloro-3-methyl-1H-pyrrolo[3,2-c]quinoline-4-carboxylic acid;
7,9-dichloro-3-methyl-1H-pyrrolo[3,2-c]quinoline-4-carboxylic acid;
7,8-dichloro-3-methyl-1H-pyrrolo[3,2-c]quinoline-4-carboxylic acid;
7-chloro-3-ethyl-1H-pyrrolo[3,2-c]quinoline-4-carboxylic acid;
7-chloro-lH-pyrrolo[3,2-c]quinoline-4-carboxylic acid;
7-fluoro-3-methyl-1H-pyrrolo[3,2-c]quinoline-4-carboxylic acid, hydrate;m.p.>300°C (dec);
8-fluoro-3-methyl-1H-pyrrolo[3,2-c]quinoline-4-carboxylic acid;
6-fluoro-3-methyl-1H-pyrrolo[3,2-c]quinoline-4-carboxylic acid;
9-fluoro-3-methyl-1H-pyrrolo[3,2-c]quinoline-4-carboxylic acid;
6,7-difluoro-3-methyl-1H-pyrrolo[3,2-c]quinoline-4-carboxylic acid;
7,9-difluoro-3-methyl-1H-pyrrolo[3,2-c]quinoline-4-carboxylic acid;
7,8-difluoro-3-methyl-1H-pyrrolo[3,2-c]quinoline-4-carboxylic acid;
7-bromo-3-methyl-1H-pyrrolo[3,2-c]quinoline-4-carboxylic acid;
6,7-dibromo-3-methyl-1H-pyrrolo[3,2-c]quinoline-4-carboxylic acid;
7,8-dibromo-3-methyl-1H-pyrrolo[3,2-c]quinoline-4-carboxylic acid;
7-nitro-3-methyl-1H-pyrrolo[3,2-c]quinoline-4-carboxylic acid;
8-nitro-3-methyl-1H-pyrrolo[3,2-c]quinoline-4-carboxylic acid 7-chloro-3-phenyl-1H-pyrrolo[3,2-c]quinoline-4-carboxylic acid; m.p.>300°c.

### Example 4

### Preparation of 7-chloro-1,3-dimethyl-1H-pyrrolo [3,2-c]quinoline-4-carboxylic acid methyl ester.

To a solution of 7-chloro-3-methyl-1H-pyrrolo [3,2-c]quinoline-4-carboxylic acid (1.2 g, 0.0046 mol) in anhydrous dimethylsulfoxide (20 ml), potassium hydroxide (0.77 g; 0.0138 mol) was added and the mixture stirred at room temperature for 1 hour. Methyl iodide (1.15 ml; 0.0185 mol) was dropped and the mixture was stirred at room temperature for 3 hours. The solution was poured into cold water and extracted with ethyl acetate. The organic layer was washed with brine, dried over anhydrous sodium sulfate, filtered and evaporated to dryness. The residue was purified by silica gel flash-chromatography (ethyl acetate as eluent) to give 0.7 g of a light brown solid (m.p. 165 °C;(dec.)); C₁₅H₁₃ClN₂O₂: required. C (62.39); H (4.54); Cl (12.28); N (9.70); found: C (62.75); H (4.73); Cl (11.98); N (9.55). Analogously the following products can be prepared :
7-fluoro-1,3-dimethyl-1H-pyrrolo[3,2-c]quinoline-4-carboxylic acid methyl ester
6,7-dichloro-1,3-dimethyl-1H-pyrrolo[3,2-c]quinoline-4-carboxylic acid methyl ester
7,8-dichloro-1,3-dimethyl-1H-pyrrolo[3,2-c]quinoline-4-carboxylic acid methyl ester
7-bromo-1,3-dimethyl-1H-pyrrolo[3,2-c]quinoline-4-carboxylic acid methyl ester
7-chloro-1-ethyl-3-methyl-1H-pyrrolo[3,2-c]quinoline-4-carboxylic acid ethyl ester

### Example 5

### Preparation of 7-chloro-1,3-dimethyl-1H-pyrrolo[3,2-c]quinoline-4-carboxylic acid.

The solution of 7-chloro-1,3-dimethyl-1H-pyrrolo[3,2-c]quinoline-4-carboxylic acid methyl ester (3.2 g; 0.011 mol) in acetic acid (15 ml) and 23% hydrochloric acid (15 ml) was refluxed for 16 hours. The solution was evaporated to dryness, taken up with water and basified with 2N sodium hydroxide. The basic solution was extracted with ethyl acetate, acidified with 2N hydrochloric acid at 0°C and the light yellow solid was collected by filtration (2.7 g; m.p. 195°C (dec.)); C₁₄H₁₁ClN₂O₂: required C (61.21); H (4.04); Cl (12.91); N (10.21); found: C (59.83); H (4.03); Cl (13.15); N (9.89).

Analogously the following products can be prepared :
6,7-dichloro-1,3-dimethyl-1H-pyrrolo[3,2-c]quinoline-4-carboxylic acid;
7,8-dichloro-1,3-dimethyl-1H-pyrrolo[3,2-c]quinoline-4-carboxylic acid;
7-fluoro-1,3-dimethyl-1H-pyrrolo[3,2-c]quinoline-4-carboxylic acid;
7-bromo-1,3-dimethyl-1H-pyrrolo[3,2-c]quinoline-4-carboxylic acid;
7-chloro-1-ethyl-3-methyl-1H-pyrrolo[3,2-c]quinoline-4-carboxylic acid;

### Example 6

Capsule, each weighing 0.23 g and containing 50 mg of the active substance can be prepared as follows:
Composition for 500 capsules:
7-chloro-3-methyl-1H-pyrrolo[3,2-c]quinoline-4-carboxylic

| | |
|---|---|
| acid | 25 g |
| Lactose | 80 g |
| Corn starch | 5 g |
| Magnesium stearate | 5 g |

This formulation can be incapsulated in two hard gelatin capsules of two pieces, each with each capsule weighing 0.23 g.

### Example 7

### Intramuscular injection of 50 mg/ml

A pharmaceutical injectable composition can be manifactured dissolving 50 g of 7-chloro-3-methyl-1H-pyrrolo[3,2-c]quinoline-4-carboxylic acid in sterile propyleneglycol (1000 ml) and sealed in 1-5 ml ampoules.

## Claims

1. A compound of formula I: 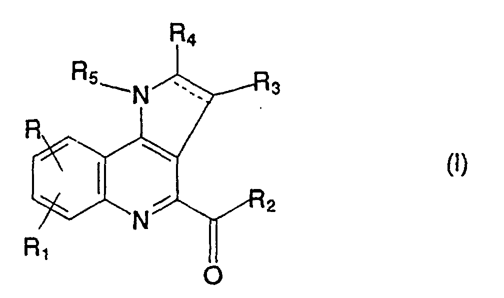 wherein
each of R and R₁, which are the same or different, is hydrogen, halogen, hydroxy, trifluoromethyl, cyano, nitro, phenyl, benzyl, C₁-C₆ alkyl, C₁-C₆ alkoxy, C₁-C₆ alkylthio, SOR₆ , SO₂R₆ , SO₂NH₂, formyl, C₂-C₆ alkanoyl, carboxy, C₁-C₆ alkoxycarbonyl or a -N(R₇R₈) group in which each of R₇ and R₈ independently is hydrogen, C₁-C₆ alkyl, formyl or C₂-C₆ alkanoyl;
R₂ is hydroxy, C₁-C₆ alkoxy, -N(R₉R₁₀) in which each of R₉ and
R₁₀, which are the same or different, is hydrogen, C₁-C₆ alkyl or phenyl, or NHOR₁₁ wherein R₁₁ is hydrogen, C₁-C₆ alkyl, benzyl or phenyl;
each of R₃, R₄ and R₆, which are the same or different is hydrogen, C₁-C₆ alkyl benzyl or phenyl; R₅ is hydrogen, C₁-C₆ alkyl, benzyl, C₂-C₆ alkanoyl, C₂-C₄ alkenyl, C₂-C₄ alkynyl or formyl; and ---- represents a double or single bond; or a pharmaceutically or veterinarily acceptable salt thereof;

2. A compound according to claim 1 wherein each of R and R₁, being the same or different, is hydrogen or halogen atom, hydroxy, trifluoromethyl, cyano, nitro, C₁-C₄ alkyl, C₁-C₄ alkoxy, C₁-C₄ alkylthio, SOR₆, SO₂R₆, SO₂NH₂, formyl, C₂-C₆ alkanoyl, carboxy, C₁-C₆ alkoxycarbonyl or -N(R₇R₈) group in which each of R₇ and R₈, which are the same or different, is hydrogen, C₁-C₄ alkyl or formyl;
R₂ is hydroxy, C₁-C₄ alkoxy, -N(R₉R₁₀) in which each of R₉ and R₁₀, which may be the same or different, is hydrogen, C₁-C₄ alkyl or phenyl, or NHOR₁₁, wherein R₁₁ is hydrogen, C₁-C₄ alkyl, benzyl or phenyl;
each of R₃, R₄ and R₆, which are the same or different, is hydrogen, C₁-C₄ alkyl or benzyl;
R₅ is hydrogen or C₁-C₄ alkyl; and
the symbol ----- represents a double bond; or a pharmaceutically or veterinarily acceptable salt thereof.

3. A process for producing a compound of formula (I) as defined in claim 1, or a pharmaceutically or veterinarily acceptable salt thereof, which process comprises hydrolysing of a compound of formula (II) 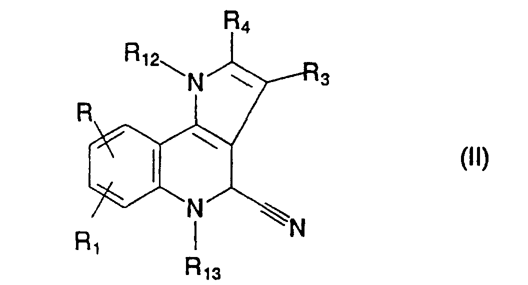 wherein R, R₁, R₃ and R₄ are as defined in claim 1, R₁₂ is C₂-C₄ alkanoyl and R₁₃ is a C₂-C₄ alkanoyl or benzoyl group, and, if desired, converting a compound of formula (I) into another compound of formula (I), and/or converting a compound of formula (I) into a pharmaceutically acceptable salt thereof, and/or, if desired, converting a salt into a free compound.

4. A process according to claim 3 wherein the hydrolysis is performed in an aqueous solution of a halohydric acid, in a suitable solvent at a temperature from 90°C to reflux.

5. A compound of formula II: 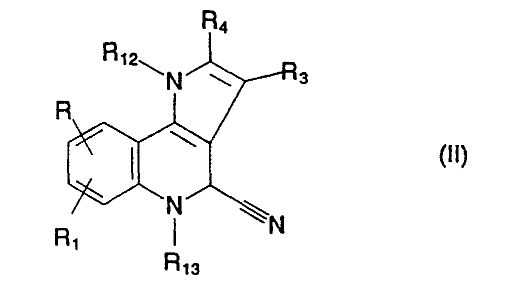 wherein R, R₁, R₃ and R₄ are as defined in claim 1, R₁₂ is C₂-C₄ alkanoyl and R₁₃ is a C₂-C₄ alkanoyl or benzoyl group.

6. A process for producing a compound of formula (II), as defined in claim 5, which process comprises treating a compound of formula III 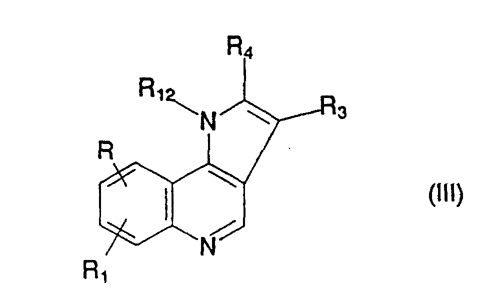 wherein R, R₁, R₃, R₄ and R₁₂ are as defined in claim 5, with sodium or potassium cyanide and an acyl halide.

7. A process according to claim 6 wherein the reaction is carried out in a mixture of solvents at a temperature from 0°C to room temperature.

8. A process for producing a compound of formula I, as defined in claim 1, which process comprises
(i) converting a compound of formula III, as defined in claim 6, to its N-oxide derivative;
(ii) treating the resulting N-oxide derivative with an excess of trimethylsilyl cyanide in the presence of a base; and
(iii) hydrolyising the compound produced in step (ii).

9. A composition comprising a pharmaceutically or veterinarily acceptable carrier and/or diluent and, as an active principle, a compound as defined in claim 1 or 2 or a pharmaceutically or veterinarily acceptable salt thereof.

10. A compound of formula (I) as defined in claim 1 or a pharmaceutically or veterinarily acceptable salt thereof, for use in the prevention and/or treatment of cerebral ischemia/hypoxia, Parkinson's disease, epilepsy, Huntington's chorea, Alzheimer's or non-Alzheimer's dementia, head or spinal cord injury, Amyotrophic Lateral Sclerosis, glaucoma/retinopathy, or infections or inflammations of the brain.

## Patentansprüche

1. Verbindung der Formel (I): 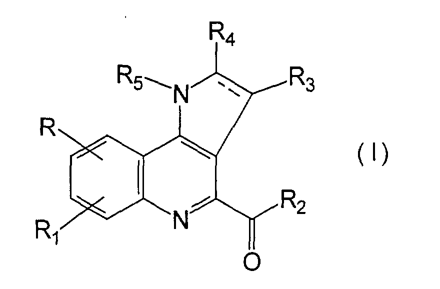 worin
jedes von R und R₁, die gleich oder verschieden sein können, ist: Wasserstoff, Halogen, Hydroxy, Trifluormethyl, Cyano, Nitro, Phenyl, Benzyl, C₁₋₆-Alkyl, C₁₋₆-Alkoxy, C₁₋₆-Alkylthio, SOR₆, SO₂R₆, SO₂NH₂, Formyl, C₂₋₆-Alkanoyl, Carboxy, C₁₋₆-Alkoxycarbonyl oder eine -N(R₇R₈)-Gruppe, bei der jeweils R₇ und R₈ unabhängig sein können: Wasserstoff, C₁₋₆-Alkyl, Formyl oder C₂₋₆-Alkanoyl;
R₂ ist Hydroxy, C₁₋₆-Alkoxy, -N(R₉R₁₀), wobei jeweils R₉ und R₁₀, die gleich oder verschieden sein können, ist: Wasserstoff, C₁₋₆-Alkyl oder -Phenyl oder NHOR₁₁, wobei R₁₁ Wasserstoff, C₁₋₆-Alkyl, Benzyl oder Phenyl ist;
jedes von R₃, R₄ und R₆, die gleich oder verschieden sein können, ist: Wasserstoff, C₁₋₆-Alkyl, Benzyl oder Phenyl; R₅ ist Wasserstoff, C₁₋₆-Alkyl, Benzyl, C₂₋₆-Alkanoyl, C₂₋₄-Alkenyl, C₂₋₄-Alkinyl oder -Formyl; und --- eine Doppel- oder Einfachbindung darstellt, oder ein pharmazeutisch oder veterinärmedizinisch annehmbares Salz davon.

2. Verbindung gemäss Anspruch 1, wobei jedes von R und R₁, die gleich oder verschieden sein können, ist: ein Wasserstoff- oder Halogenatom, Hydroxy, Trifluormethyl, Cyano, Nitro, C₁₋₄-Alkyl, C₁₋₄-Alkoxy, C₁₋₄-Alkylthio, SOR₆, SO₂R₆, SO₂NH₂, Formyl, C₂₋₆-Alkanoyl, Carboxy, C₁₋₆-Alkoxycarbonyl oder eine -N(R₇R₈)-Gruppe, bei der jeweils R₇ und R₈, die gleich oder verschieden sein können, ist: ein Wasserstoff, C₁₋₄-Alkyl oder Formyl;
R₂ ist Hydroxy, C₁₋₄-Alkoxy, -N(R₉R₁₀), wobei jeweils R₉ und R₁₀, die gleich oder verschieden sein können, ein Wasserstoff, C₁₋₄-Alkyl oder Phenyl oder NHOR₁₁, worin R₁₁ ein Wasserstoff, C₁₋₄-Alkyl, Benzyl oder Phenyl ist, ist;
jedes von R₃, R₄ und R₆, die gleich oder verschieden sein können, ist ein Wasserstoff, C₁₋₄-Alkyl oder Benzyl;
R₅ ist Wasserstoff oder C₁₋₄-Alkyl; und
das Symbol --- stellt eine Doppelbindung dar; oder ein pharmazeutisch oder veterinärmedizinisch annehmbares Salz davon.

3. Verfahren zur Herstellung einer Verbindung der Formel (I) gemäss Anspruch 1 oder eines pharmazeutisch oder veterinärmedizinisch annehmbaren Salzes davon, wobei das Verfahren umfasst: Hydrolysieren einer Verbindung der Formel (II): 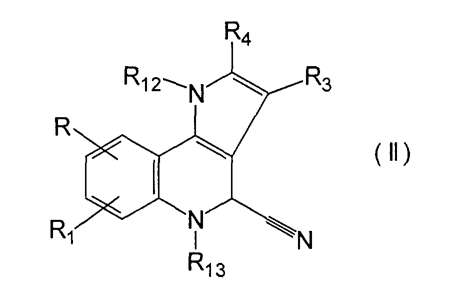 worin R, R₁, R₃ und R₄ wie in Anspruch 1 definiert sind, R₁₂ ist C₂₋₄-Alkanoyl und R₁₃ ist eine C₂₋₄-Alkanoyl- oder Benzoylgruppe und, wenn gewünscht, Umwandeln einer Verbindung der Formel (I) in eine andere Verbindung der Formel (I), und/oder Umwandeln einer Verbindung der Formel (I) in ein pharmazeutisch annehmbares Salz davon und/oder, wenn gewünscht, Umwandeln eines Salzes in eine freie Verbindung.

4. Verfahren gemäss Anspruch 3, wobei die Hydrolyse in einer wässrigen Lösung einer Halogenwasserstoffsäure in einem geeigneten Lösungsmittel bei einer Temperatur von 90°C unter Rückfluss durchgeführt wird.

5. Verbindung der Formel (II): 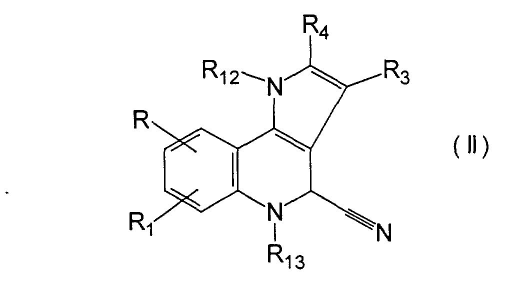 worin R, R₁, R₃ und R₄ wie in Anspruch 1 definiert sind, R₁₂ ist C₂₋₄-Alkanoyl und R₁₃ ist eine C₂₋₄-Alkanoyl- oder Benzoylgruppe.

6. Verfahren zur Herstellung einer Verbindung der Formel
(II) wie in Anspruch 5 definiert, das Verfahren umfasst das Behandeln einer Verbindung der Formel (III): 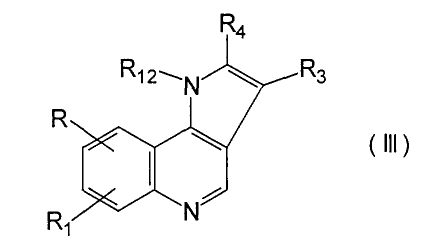 worin R, R₁, R₃, R₄ und R₁₂ wie in Anspruch 5 definiert sind, mit Natrium- oder Kaliumcyanid und einem Acylhalogenid.

7. Verfahren gemäss Anspruch 6, wobei die Reaktion in einer Mischung von Lösungsmitteln bei einer Temperatur von 0°C bis Raumtemperatur durchgeführt wird.

8. Verfahren zur Herstellung einer Verbindung der Formel
(I) wie in Anspruch 1 definiert, das Verfahren umfasst:
(i) Umwandeln einer Verbindung der Formel (III) wie in Anspruch 6 definiert in sein N-Oxidderivat;
(ii) Behandeln des erhaltenen N-Oxidderivats mit einem Überschuss an Trimethylsilylcyanid in Anwesenheit einer Base und
(iii) Hydrolysieren der Verbindung, hergestellt in Schritt (ii).

9. Zusammensetzung, umfassend einen pharmazeutisch oder veterinärmedizinisch annehmbaren Träger und/oder einen Verdünner und als Wirkstoff eine Verbindung gemäss Anspruch 1 oder 2 oder ein pharmazeutisch oder veterinärmedizinisch annehmbares Salz davon.

10. Verbindung der Formel (I) wie in Anspruch 1 definiert oder ein pharmazeutisch oder veterinärmedizinisch annehmbares Salz davon zur Verwendung bei der Vorbeugung und/oder Behandlung von cerebraler Ischämie/Hypoxie, Parkinsonerkrankung, Epilepsie, Huntington's chorea, Alzheimer oder Nicht-Alzheimer Demenz, Kopf- oder spinaler Cordaverletzung, amyotrophischer lateraler Sklerose, Glaukom/Retinopathie oder Infektionen oder Entzündungen des Gehirns.

## Revendications

1. Composé de formule I : 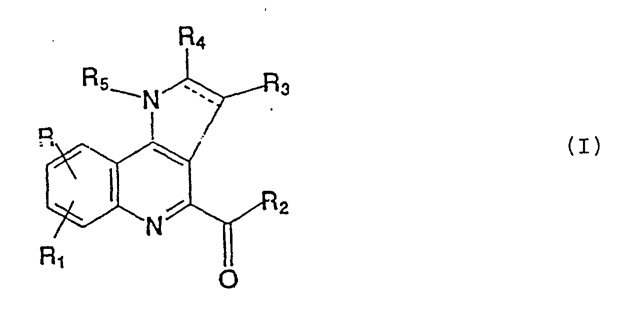 dans laquelle
R et R₁, qui sont identiques ou différents, représentent chacun hydrogène, halogène, hydroxy, trifluorométhyle, cyano, nitro, phényle, benzyle, alkyle en C₁-C₆, alkoxy en C₁-C₆, alkylthio en C₁-C₆, SOR₆, SO₂R₆, SO₂NH₂, formyle, alcanoyle en C₂-C₆, carboxy, (alkoxy en C₁-C₆)carbonyle ou un groupe -N(R₇R₈) où R₇ et R₈ représentent chacun indépendamment hydrogène, alkyle en C₁-C₆, formyle ou alcanoyle en C₂-C₆ ;
R₂ est hydroxy, alkoxy en C₁-C₆, -N(R₉R₁₀) où R₉ et R₁₀, qui sont identiques ou différents, représentent chacun hydrogène, alkyle en C₁-C₆ ou phényle, ou NHOR₁₁ où R₁₁ est hydrogène, alkyle en C₁-C₆, benzyle ou phényle ;
R₃, R₄ et R₆, qui sont identiques ou différents représentent chacun hydrogène, alkyle en C₁-C₆, benzyle ou phényle ; R₅ est hydrogène, alkyle en C₁-C₆, benzyle, alcanoyle en C₂-C₆, alcényle en C₂-C₄, alcynyle en C₂-C₄ ou formyle ; et ---- représente une double ou une simple liaison ; ou un sel de celui-ci acceptable sur le plan pharmaceutique ou vétérinaire.

2. Composé selon la revendication 1, dans lequel R et R₁, qui sont identiques ou différents, représentent chacun hydrogène ou halogène, hydroxy, trifluorométhyle, cyano, nitro, alkyle en C₁-C₄, alkoxy en C₁-C₄, alkylthio en C₁-C₄, SOR₆, SO₂R₆, SO₂NH₂, formyle, alcanoyle en C₂-C₆, carboxy, (alkoxy en C₁-C₆)carbonyle ou un groupe -N(R₇R₈) où R₇ et R₈, qui sont identiques ou différents, représentent chacun hydrogène, alkyle en C₁-C₄ ou formyle ;
R₂ est hydroxy, alkoxy en C₁-C₄, -N(R₉R₁₀) où R₉ et R₁₀, qui peuvent être identiques ou différents, représentent chacun hydrogène, alkyle en C₁-C₄ ou phényle, ou NHOR₁₁ où R₁₁ est hydrogène, alkyle en C₁-C₄, benzyle ou phényle ;
R₃, R₄ et R₆, qui sont identiques ou différents, représentent chacun hydrogène, alkyle en C₁-C₄ ou benzyle ;
R₅ est hydrogène ou alkyle en C₁-C₄ ; et
le symbole ---- représente une double liaison ; ou un sel de celui-ci acceptable sur le plan pharmaceutique ou vétérinaire.

3. Procédé de production d'un composé de formule (I) comme défini dans la revendication 1, ou d'un sel de celui-ci acceptable sur le plan pharmaceutique ou vétérinaire, lequel procédé comprend l'hydrolyse d'un composé de formule (II) 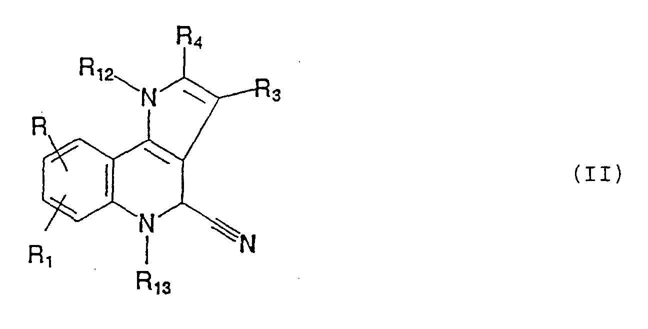 dans laquelle R, R₁, R₃ et R₄ sont comme définis dans la revendication 1, R₁₂ est alcanoyle en C₂-C₄ et R₁₃ est alcanoyle en C₂-C₄ ou groupe benzoyle, et, si souhaité, la transformation d'un composé de formule (I) en un autre composé de formule (I), et / ou la transformation d'un composé de formule (I) en un sel pharmaceutiquement acceptable de celui-ci, et / ou , si souhaité, la transformation d'un sel en un composé libre.

4. Procédé selon la revendication 3, dans lequel l'hydrolyse est effectuée dans une solution aqueuse d'un acide halohydrique, dans un solvant approprié à une température de 90°C à la température de reflux.

5. Composé de formule II : 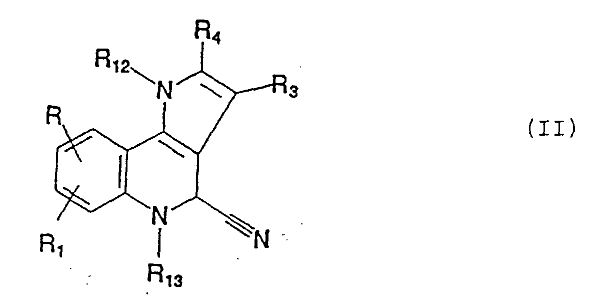 dans laquelle R, R₁, R₃ et R₄ sont comme définis dans la revendication 1, R₁₂ est alcanoyle en C₂-C₄ et R₁₃ est alcanoyle en C₂-C₄ ou groupe benzoyle.

6. Procédé de production d'un composé de formule (II), comme défini dans la revendication 5, lequel procédé comprend le traitement d'un composé de formule III 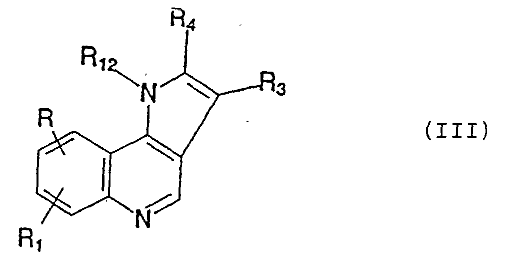 dans laquelle R, R₁, R₃, R₄ et R₁₂ sont comme définis dans la revendication 5, avec du cyanure de sodium ou de potassium et un halogénure d'acyle.

7. Procédé selon la revendication 6, dans lequel la réaction est effectuée dans un mélange de solvants à une température de 0°C à la température ambiante.

8. Procédé de production d'un composé de formule I, comme défini dans la revendication 1, lequel procédé comprend
(i) la transformation d'un composé de formule III, comme défini dans la revendication 6, en son dérivé N-oxyde ;
(ii) le traitement du dérivé N-oxyde résultant avec un excès de cyanure de triméthylsilyle en présence d'une base ; et
(iii) l'hydrolyse du composé produit dans l'étape (ii).

9. Composition comprenant un véhicule et / ou un diluant acceptable sur le plan pharmaceutique ou vétérinaire et, en tant que principe actif, un composé comme défini dans la revendication 1 ou 2 ou un sel de celui-ci acceptable sur le plan pharmaceutique ou vétérinaire.

10. Composé de formule (I) comme défini dans la revendication 1 ou un sel de celui-ci acceptable sur le plan pharmaceutique ou vétérinaire, pour utilisation dans la prévention et / ou le traitement de l'ischémie/hypoxie cérébrale, de la maladie de Parkinson, de l'épilepsie, de la chorée de Huntington, de la démence d'Alzheimer ou non, d'une blessure à la tête ou à la moelle épinière, de la Sclérose Amyotrophique Latérale, du glaucome/de la rétinopathie ou des infections ou des inflammations du cerveau.
